# EUROPEAN PATENT APPLICATION

(11) **EP 4 703 345 A1**
(43) Date of publication of application: **04.03.2026**
(21) Application number: 24196330.5
(22) Date of filing: 25.08.2024
(51) Int. Cl.: C07C 67/30, C07C 69/58

(54) **A PROCESS FOR THE SELECTIVE DEOXYGENATION OF CARBOXYLIC ACIDS OR DERIVATIVES THEREOF**

(71) Applicant: Universität Konstanz, 78464 Konstanz (DE)
(72) Inventor: Häußler, Manuel, 14482 Potsdam (DE); Odenwald, Lukas, 78464 Konstanz (DE); Race, James, 78464 Konstanz (DE)
(74) Representative: Deblon, Jörg-Stephan

(57) **Abstract**

The present invention relates to a process for preparing carboxylic acid compounds such as mono- and α,ω-dicarboxylic acid compounds by reacting carboxylic acid compounds comprising at least one hydroxy or oxo-group with hydrogen or a hydrogen carrier in the presence of at least a hydrogenation catalyst comprising a transition metal or a salt thereof and at least one dehydration catalyst either sequentially or simultaneously.

## Description

The present invention relates to a process for preparing carboxylic acid compounds such as mono- and α,ω-dicarboxylic acid compounds by reacting carboxylic acid compounds comprising at least one hydroxy or oxo-group with hydrogen or a hydrogen carrier in the presence of at least one hydrogenation catalyst comprising a transition metal or a salt thereof and at least one dehydration catalyst either sequentially or simultaneously.

Carboxylic acids with 8 to 50 carbon atoms, in particular saturated mono- and α,ω-dicarboxylic acids, are key platform chemicals inter alia for the manufacture of surfactants, cosmetics, paints, coatings, lubricants, adhesives, polymers and various other products used in daily life.

Due to their natural abundance, straight chained monocarboxylic acids preferably with an even number of 8 to 24 carbon atoms, also referred to as fatty acids, are typically obtained from oils such as rapeseed, palm, palm kernel, coconut, soy bean, castor and sunflower oil as well as from used cooking oils. While hydrogenation of mono- or polyunsaturated fatty acids can be achieved by various known methods in good yields, further functionalization to obtain α,ω-dicarboxylic acids i.e. via oxidative cleavage remain a challenge. The publication of U. Biermann et al., "Fatty Acids and their Derivatives as Renewable Platform Molecules for the Chemical Industry", Angew. Chem. Int. Ed. 2021, 60, 20144- 20165 summarizes various chemical, and biochemical approaches therefor.

As vegetable oils are frequently associated with deforestation of rain forests in particular in developing countries sustainability concerns made it desirable to seek alternative resources for the production of carboxylic acids.

In a recent approach disclosed in WO2023/118135A carboxylic acids in particular saturated α,ω-dicarboxylic acids could be produced by catalytic oxidative cleavage from (waste) polyethylene. As a side product oxygenated compounds comprising hydroxy- and oxo-groups were observed.

Some natural oils and due to their exposure to air at higher temperature used cooking oils also contain hydroxy- and oxo-groups which are typically undesired in above mentioned applications. Their selective removal while preserving the carboxylic acid functionality still remains a challenge.

Therefore, there was still a need to provide an efficient process to selectively remove hydroxy- or oxo-groups from carboxylic acids comprising the same.

A process has now been found for the preparation of compounds of formula (I)

[EG-R-COO]ₙA (I),

wherein
n is 1, 2 or 3, preferably 1 or 3, more preferably 1,
   A is
   for n=1 hydrogen or C₁-C₁₂-alkyl, preferably hydrogen or C₁-C₄-alkyl, more preferably hydrogen, methyl or ethyl,
   for n=2 C₂-C₆-alkanediyl, preferably 1,2-ethandiyl and
   for n=3 1,2,3-propanetriyl
EG is an end group selected from COOE, CH=O, CH₂-OH and CH₃, preferably selected from COOE, CH₂-OH and CH₃ and in one embodiment from COOE and CH₂-OH wherein E is hydrogen or C₁-C₁₂-alkyl, preferably hydrogen or C₁-C₄-alkyl, more preferably hydrogen, methyl or ethyl
R is C₆-C₄₈-alkanediyl, preferably C₆-C₄₀-alkanediyl and even more preferably C₆-C₃₂-alkanediyl
the process comprising at least the steps of reacting compounds of formula (II)

   [EG-R_{OX}-COO]ₙA (II),

   wherein n, A and EG have the meaning defined above for formula (I) including their preferences and
   Rox is C₆-C₄₈-alkanediyl or C₆-C₄₈-alkenediyl, which is once or more substituted by substituents selected from hydroxy and/or oxo, preferably C₆-C₄₀-alkanediyl or C₆-C₄₀-alkenediyl, which is once or more substituted by substituents selected from hydroxy and/or oxo and even more preferably C₆-C₃₂-alkanediyl or C₆-C₃₂-alkenediyl which is once or more substituted by substituents selected from hydroxy and/or oxo
   either sequentially or simultaneously, preferably simultaneously
      - with molecular hydrogen (H₂) or a hydrogen carrier in the presence of at least one hydrogenation catalyst comprising a transition metal or a salt thereof whereby the transition metal is selected from Ni, Cu, Mo, Cr, W, Ru, Rh, Pt, Pd and Ir and
      - in the presence of at least one dehydration catalyst.

The scope of the invention encompasses all combinations of substituent definitions, parameters and illustrations set forth above and below, either in general or within areas of preference or preferred embodiments, with one another, i.e., also any combinations between the particular areas and areas of preference.

Whenever used herein the terms "including", "e.g.", "such as" and "like" are meant in the sense of "including but without being limited to" or "for example without limitation", respectively.

As used herein, and unless specifically stated otherwise **C₆-C₄₈-alkanediyl** denotes an alkanediyl substituent with 6 to 48 carbon atoms which may be either acyclic or cyclic either in part or as a whole, branched or unbranched, preferably acyclic and branched or unbranched even more preferably acyclic and unbranched. This definition analogously applies to **C₁-C₅-alkanediyl** with 1 to 5 carbon atoms, **C₆-C₄₀**-**alkaned**with 6 to 40 carbon atoms, **C₆-C₃₂-alkanediyl** with 6 to 32 carbon atoms and **C₄₉-C₁₀₀-alkanediyl** with 49 to 100 carbon atoms.

As used herein, and unless specifically stated otherwise **C₆-C₄₈-alkenediyl** denotes a hydrocarbon substituent with 6 to 48 carbon atoms which may be either acyclic or cyclic either in part or as a whole, branched or unbranched, preferably acyclic and branched or unbranched even more preferably acyclic and unbranched and comprising one or more, preferably one, two or three, for example one or two non-adjacent carbon-carbon double bonds. This definition analogously applies to **C₁-C₅-alkenediyl** with 1 to 5 carbon atoms, **C₆-C₄₀-alkenediyl** with 6 to 40 carbon atoms, **C₆-C₃₂-alkanediyl** with 6 to 32 carbon atoms and **C₄₉-C₁₀₀-alkenediyl** with 49 to 100 carbon atoms.

As used herein, and unless specifically stated otherwise **C₁-C₁₂-alkyl** denotes an alkyl substituent with 1 to 12 carbon atoms which may be either acyclic or for 5-12 carbon atoms cyclic either in part or as a whole, branched or unbranched, preferably acyclic and branched or unbranched even more preferably acyclic and unbranched.

Specific C₁-C₁₂-alkyl-substituents include methyl, ethyl, n-propyl, n-butyl, tert.- butyl, isobutyl, n-pentyl, cyclopentyl, n-hexyl, cyclohexyl, 2-ethylhexyl, n-octyl, n-decyl and n-dodecyl.

As used herein, and unless specifically stated otherwise C₁-C₄-alkyl denotes methyl, ethyl, isopropyl, n-propyl, n-butyl, isobutyl and tert.-butyl, preferably methyl and ethyl.

Examples for R include n-hexandiyl, n-heptandiyl, n-octandiyl, n-nonandiyl, n-decanediyl, n-undecanediyl, n-dodecanediyl, n-tridecanediyl, n-tetradecanediyl, n-pentadecanediyl, n-hexadecanediyl, n-heptadecanediyl, n-octadecanediyl, n-nonadecanediyl, n-eicosanediyl, n-heneicosanediyl, n-doeicosanediyl, n-tricosanediyl, n-tetracosanediyl, n-pentycosanediyl, n-hexacosanediyl, n-heptacosanediyl, n-octacosanediyl, n-nonacosanediyl, n-tricontanediyl, n-hentricontanediyl, n-dotriacontanediyl, n-tritriacontanediyl, n-tetratriacontanediyl, n-hexatetracontanediyl.

Examples of compounds of formula (I) include n-octanecarboxylic acid, 1,8-octanedicarboxylic acid, n-nonanecarboxylic acid, 1,9-nonanedicarboxylic acid, n-decanecarboxylic acid, 1,10-decanedicarboxylic acid, 1,11-undecanedicarboxylic acid, n-undecancarboxylic acid, n-dodecanecarboxylic acid, 1,12-dodecanedicarboxylic acid, n-tridecanecarboxylic acid, 1,13-tridecanedicarboxylic acid, n-tetradecanecarboxylic acid, 1,14-tetradecanedicarboxylic acid, n-pentadecanecarboxylic acid, 1,15-pentadecanedicarboxylic acid, n-hexadecanecarboxylic acid, 1,16-hexadecanedicarboxylic acid, n-heptadecanecarboxylic acid, 1,17-heptadecanedicarboxylic acid, n-octadecanecarboxylic acid, 1,18-octadecanedicarboxylic acid, n-nonadecanecarboxylic acid, 1,19-nonadecanedicarboxylic acid, n-eicosaneanecarboxylic acid, 1,20-eicosanedicarboxylic acid, n-heneicosanecarboxylic acid, 1,21-heneicosanedicarboxylic acid, n-docosanecarboxylic acid, 1,22-docosanedicarboxylic acid, n-tricosanecarboxylic acid, 1,23-tricosanedicarboxylic acid, n-tetracosanecarboxylic acid, 1,24-tetracosanedicarboxylic acid, n-pentacosanecarboxylic acid, 1,25-pentacosanedicarboxylic acid, n-hexacosanecarboxylic acid, 1,26-hexacosanedicarboxylic acid, n-heptacosanecarboxylic acid, 1,27-heptacosanedicarboxylic acid, n-octacosanecarboxylic acid 1,28-octacosanedicarboxylic acid, n-nonacosanecarboxylic acid, 1,29-nonacosanedicarboxylic acid, n-tricontanecarboxylic acid, 1,30-tricontanedicarboxylic acid, n-hentricontanecarboxylic acid, 1,31-hentricontanedicarboxylic acid, n-dotriacontanecarboxylic acid, 1,32-dotriacontanedicarboxylic acid, n-tritriacontanecarboxylic acid, 1,33-tritriacontanedicarboxylic acid, n-tetratriacontanecarboxylic acid, 1,34-tetratriacontanedicarboxylic acid, n-dotetracontanecarboxylic acid, 1,42-dotetracontanedicarboxylic acid, n-pentacontanecarboxylic acid, 1,50-pentacontanedicarboxylic acid as well as the methyl and ethyl esters of all of the aforementioned carboxylic acids.

Exemplary compounds of formula (II) are methyl 12-ketooctadecanoate, 16-hydroxy palmitic acid and (9Z,12R)-12-hydroxy octadec-9-enoic acid.

In a specific embodiment the process according to the invention is used to produce specific compounds of formula (I) i.e. those of formula (Ia)

[EOOC-R-COO]ₙA (Ia),

wherein R, E, n and A have the meaning as defined above for formula (I) including their respective preferences
by reacting specific compounds of formula (II) i.e. those of formula (IIa)

   [EOOC-R_{OX}-COO]ₙA (IIa),
wherein Rox has the meaning as defined above for formula (I) including its preferences.

In one embodiment the starting material employed in the process according to the invention comprises mixtures of at least two, preferably at least five and more preferably at least ten different compounds of formula (II).

In one embodiment the starting material employed in the process according to the invention comprises mixtures of compounds of formula (II) having at least two, preferably at least five and more preferably at least ten, for example ten to forty-three different numbers of carbon atoms.

The compounds of formula (II) may be employed in neat form or in a solvent that is inert or virtually inert under the reaction conditions i.e. with regard to dehydration and/or hydrogenation.

Depending on the source of compounds of formula (II) the starting material may further contain compounds of formula (I), whereby the latter would then also act as a solvent.

Since the definition of compounds of formula (II) include compounds of formula (IIa) the above embodiments consequently apply to those compounds of formula (IIa) as well.

Suitable solvents include C₁-C₁₂-n-alkanols such as methanol and ethanol, whereby methanol is preferred.

The weight ratio of solvent to compounds of formula (II) if employed or present at the beginning of the process according to the invention is typically from 0.5 to 100, preferably from 1 to 100 and more preferably from 5 to 50.

It is apparent to those skilled in the art that during the performance of the process according to the invention the content of compounds of formula (I) increases and thus the amount of compounds that may act as a solvent.

Besides the compounds of formula (II) employed in the inventive process and depending on the source of compounds of formula (II) optionally also compounds of formula (I) the starting materials may further contain compounds of formula (III)

[EG-R_{EXCL}-COO]ₙA (III),

wherein
EG, n and A have the meaning as defined for formula (II) above including their preferences
R_{EXCL} is C₁-C₅-alkanediyl or C₁-C₅-alkenediyl or C₄₉-C₁₀₀-alkanediyl or C₄₉-C₁₀₀-alkenediyl which is not, once or more substituted by substituents selected from hydroxy and/or oxo.

In particular in cases where mixtures of formula (IIa) are employed as starting materials which may be products obtained from oxidative cleavage of polyethylene the reaction mixture may further contain compounds of formula (IIIa)

[EOOC-R_{EXCL}-COO]ₙA (IIIa)

wherein R_{EXCL} has the meaning as defined above for formula (III)

In these embodiments, the amount of compounds of formula (III) present at the beginning of the process according to the invention is typically from 0.01 to 20 wt.-%, preferably from 0.1 to 15 wt.-% and more preferably from 0.1 to 5 wt.-%.

It is apparent to those skilled in the art that during the performance of the process according to the invention the content of compounds of formula (III) might decrease due to hydrogenation or dehydration reactions. Its noteworthy that the products obtained from compounds of formula (III) where R_{EXCL} is C₁-C₅-alkanediyl or C₁-C₅-alkenediyl may be much different compared to those obtained for compounds of formula (II), see also in the experimental part.

In a specific embodiment the mixture of compounds of formula (IIa) used as starting material for the process according to the invention is prepared by at least the step of
a) reacting polyethylene having a number-average molecular weight Mₙ of at least 20,000 g/mol with an oxygen-containing gas at a temperature of from the melting point of the polyethylene to 300°C in the presence of an oxidation catalyst, for example an oxidation catalyst that is insoluble in the reaction mixture.

In this embodiment the polyethylene may be applied as a polyethylene containing mixture having a polyethylene content of at least 50% by mass.

Polyethylene may include LLDPE, LDPE and HDPE or mixtures thereof. It may be virgin material, production cuttings or residues or may stem form secondary raw material streams.

The remainder to 100 % may be additives or foreign materials as typically found in secondary raw material streams as they occur in the plastic waste management industry for example in Germany or Europe and is defined and also traded for example by various national standards (e.g. green dot in Germany) and provided that these do not prevent the oxidative degradation of polyethylene with oxygen. Such further additives and foreign materials include liquid and solid plasticizers, stabilizers, lubricants and mould release agents, compatibilizers, nucleating agents, fibrous or non-fibrous fillers, dyes, solvent residues, food residues, general product residues (e.g. shampoo), glass, ceramics, rubber, stones, wood, textiles, paper, cardboard, paperboard, metal foils, polymers such as polyethers, polyesters, polyamides, polyurethanes, polycarbonates, polypropylene, polystyrene, polyvinylchloride or combinations thereof.

Suitable oxygenation catalysts in step a) include N-hydroxyphthalimide as well as transition metals whereby the transition metals are preferably selected from the group consisting of Mo, Ru, Rh, Pd, Ag, W, Re, Os, Ir, Pt and Au, or transition metal compounds whereby the transition metal compounds are preferably selected from compounds of Mn, Fe, Co, Ni, Cu, Zn, Cr, V, Ti, Ru, Rh, Pd, Ag, Mo, W, Re, Os, Ir, Pt and Au in form of naphthenates, acetates, acetyl acetonates, chlorides, bromides, iodides, nitrates, oxide, sulphates, carbonates, phosphates and C₆-C₂₄ carboxylates such as laurate, myristate, palmitate, palmitoleate, stearate, oleate and erucate.or mixtures of any of the aforementioned catalysts.

N-hydroxyphthalimide and manganese dioxide or mixtures thereof are preferred, whereby manganese dioxide is even more preferred.

Typically the amount of the at least one oxygenation catalyst is 0.0001 to 300.0 % by mass, based on 100% by mass of the polyethylene-containing mixture employed in step a).

In one embodiment step a) action is carried out in the presence of water.

In this embodiment the amount of water may be in the range of 0.1 to 200 wt-% of the polyethylene employed. Larger amounts are possible but do not add an advantage.

Step a) can be for example, performed at a temperature of 140 to 300°C, preferably 140 to 250°C.

The partial pressure of the oxygen-containing gas in step a) is typically from 100 hPa to 10 MPa, preferably from 0.1 MPa to 10 MPa, more preferably from 0.1 MPa to 5 MPa and yet even more preferably from 0.5 MPa to 3 MPa.

The oxygen-containing gas contains for example 5.0 to 100.0 % of oxygen by volume the remainder, if any, being gases inert under the reaction conditions such as nitrogen and/or noble gases and/or carbon dioxide. Preferably, the oxygen-containing gas contains 15.0 to 100.0 % of oxygen. Examples include synthetic air (20.0% by volume oxygen and 80.0% by volume nitrogen) or atmospheric air.

As used herein oxygen denotes ozone (O₃) and dioxygen (O₂), in one embodiment dioxygen (O₂) is employed in the oxygen-containing gas. In a further embodiment no ozone is present.

According to the invention, the reaction time is for example from 0.1 to 24 hours, preferably from 1 to 8 hours.

In one embodiment the process to prepare a mixture of compounds of formula (IIa) further contains the separation of the reaction products from the reaction mixture and/or their purification. This can for example be done in a manner known to those skilled in the art.

Such separation and/or purification steps may encompass at least one process selected from the group consisting of filtration, centrifugation, sedimentation, drying, precipitation, extraction, crystallization, distillation and chromatographic processes.

The process according to the invention to selectively remove hydroxyl- or oxo-groups from carboxylic acids comprises the sequential or simultaneous reaction with molecular hydrogen (H₂) in the presence of at least one hydrogenation catalyst and at least one dehydration catalyst.

Without wanting to be bound by theory the reaction presumably proceeds via two or three steps. First, if present in the starting materials of formula (II), the hydrogenation of oxo-groups to hydroxyl groups, then the dehydration of hydroxy groups to alkene groups and finally the hydrogenation of alkene groups to the desired saturated compounds of formula (I).

If the process according to the invention is carried out sequentially, the compounds of formula (II) are in a first step
i) reacted with hydrogen in the presence of at least one hydrogenation catalyst and then ii) dehydrated in the presence of a dehydration catalyst and finally
iii) the resulting alkene containing products obtained in step ii) are reacted with hydrogen in the presence of at least one hydrogenation catalyst to obtain the desired products of formula (I).

Where the products of formula (II) do not contain oxo groups step i) may be omitted.

It is, however, a major finding of the invention that steps i) to iii) may be performed simultaneously in the same reaction vessel.

The process according to the invention can be performed, for example, in any reaction vessel known to those skilled in the art for hydrogenations and dehydrations e.g. in a flow-through reactor, a fixed bed reactor, a trickle flow reactor e.g. a trickle film or trickle bed reactor, a fluidized bed reactor or a suspension reactor for example a bubble column reactor or a stirred reactor with gas inlet. In a preferred embodiment the reaction is performed in a stirred reactor with gas inlet.

The process according to the invention is performed in the presence of at least one hydrogenation catalyst comprising a transition metal or a salt thereof whereby the transition metal is selected from Ru, Rh, Pt, Pd, Ir, Ni, Mo, Cr, Cu and W, including porous metal catalysts such as Raney-nickel, Raney-cobalt and Urushibara type catalysts like Urushibara-Nickel.

Such hydrogenation catalysts include those wherein the transition metal or a salt thereof is supported on a carrier material such as zirconium dioxide, titanium dioxide, silicates such as magnesium or aluminium silicates, aluminium oxides, silicon dioxide, graphite, activated carbons or alkaline earth metal compounds as well as on mixtures of these carrier materials.

The content of transition metal or a salt thereof of the supported hydrogenation catalyst is typically from 0.1 to 20, preferably from 0.5 to 10 wt-% calculated on the transition metal content.

The shape of the hydrogenation catalysts is not particularly limited, and the hydrogenation catalysts may be in the form of sphere, cylindrical column, extrudate or crushed particles. The size of the particles of the hydrogenation catalysts may be selected in the range of 0.01 mm to 100 mm depending on the size and type of the reaction vessel employed to perform the process according to the invention. When the hydrogenation catalyst is supported on a carrier material, the particle size of the supported catalyst is preferably in the above range.

The process according to the invention is performed in the presence of at least one dehydration catalyst. Suitable dehydration catalysts include for example metal oxide catalysts of chromium, molybdenum or tumgsten. They further include zeolites, aluminas and heteropoly acid salts wherein the protons in the heteropoly acids are partially or fully exchanged with metal cations. The dehydration catalysts may be used alone or in combination.

Zeolites are inorganic crystalline porous compounds mainly composed of silicon and aluminum and are suitable dehydration catalysts from the viewpoints of heat resistance and acid strength. Suitable zeolites may be selected appropriately inter alia depending on the molecular diameter of the compounds of formula (II).

In detail, zeolites having an eight to sixteen-membered oxygen ring pore are preferably used.

Examples of the zeolites having an eight to sixteen-membered oxygen ring pore include chabazite, erionite, ferrierite, heulandite, ZSM-5, ZSM-11, ZSM-12, NU-87, theta-1, weinbergerite, X-type zeolite, Y-type zeolite, USY-type zeolite, mordenite, dealuminated mordenite, β-zeolite, MCM-22, MCM-36, MCM-56, gmelinite, offretite, cloverite, VPT-5 and UTD-1.

Of the zeolites having an eight to twelve-membered oxygen ring pore are more preferred. Examples of the zeolites having an eight to twelve-membered oxygen ring pore include chabazite, erionite, Y-type zeolite, USY-type zeolite, mordenite, dealuminated mordenite, β-zeolite, MCM-22, MCM-56, ZSM-12 and ZSM-5. In the zeolites, the composition ratio between silicon and aluminum (silicon/aluminum) is in the range of 2/1 to 200/1, and in view of activity and heat stability, preferably in the range of 5/1 to 100/1. Further, isomorphously substituted zeolites may be used in which aluminum atoms in the zeolite skeleton are substituted with other metal such as Ga, Ti, Fe, Mn or B.

Examples of aluminas include α-alumina and γ-alumina, whereby γ-alumina is preferably used from the viewpoints of heat resistance and acid strength of the dehydration catalyst.

Examples of heteropoly acids include phosphotungstic acid, silicotungstic acid, phosphomolybdic acid and silicomolybdic acid. These heteropoly acids are commercially available. The protons in these heteropoly acids are partially or fully exchanged with metal cations. Preferred metal cations are alkali metal cations and alkaline earth metal cations. The alkali metal cations are more preferable, and potassium cations and cesium cations are particularly preferable.

Specific examples of the heteropoly acid salts include potassium phosphotungstate, potassium silicotungstate, potassium phosphomolybdate, potassium silicomolybdate, cesium phosphotungstate, cesium silicotungstate, cesium phosphomolybdate and cesium silicomolybdate.

The heteropoly acid salt may be supported on a carrier. Examples of suitable carriers are the same as mentioned above for the hydrogenation catalysts.

The shape of the dehydration catalysts is not particularly limited, and the dehydration catalysts may be in the form of sphere, cylindrical column, extrudate or crushed particles. The size of the particles of the dehydration catalysts may be selected in the range of 0.01 mm to 100 mm depending on the size and type of the reaction vessel employed to perform the process according to the invention. When the dehydration catalyst is supported on the carrier, the particle size of the supported catalyst is preferably in the above range.

There are examples of catalysts that combine both, the hydrogenation and the dehydration activity.

Suitable examples include Ruthenium on H-ZSMS; Nickel and Cobalt on H-ZSMS (see also Nagu et al., Chemistry Select, Volume 8, Issue20, 2023) as well as PtWO₃ supported on alumina (see Bhanuchander et al, New J. Chem., 2019,43, 18003-18011)

The process according to the invention can be performed batchwise or continuously, preference being given to batchwise performance.

In batchwise processes, the weight ratio of compounds of formula (II) to the hydrogenation catalyst is typically from 5 to 1,000, preferably from 10 to 100.

In batchwise processes, the weight ratio of compounds of formula (II) to the dehydration catalyst is typically from 5 to 1,000, preferably from 10 to 100.

The reaction times are typically from 2 minutes to 24 hours, preferably from 1 hour to 12 hours for each step, whether the process is performed in sequential steps or simultaneously in one step.

In continuous processes, the throughput is typically selected such that the weight ratio of compounds of formula (II) to the hydrogenation catalyst is typically from 20 to 5,000, preferably from 50 to 1,000 per hour.

In continuous processes, the throughput is typically selected such that the weight ratio of compounds of formula (II) to the dehydration catalyst is typically from 20 to 5,000, preferably from 500 to 1,000 per hour.

The residence times are for example from 1 minute to 3 hours, preferably from 3 minutes to 60 minutes for each step, whether the process is performed in sequential steps or simultaneously in one step.

The process according to the invention is performed, for example, at a reaction temperature of 20 to 280°C, preferably 100 to 250°C, more preferably 180 to 220 °C.

The compounds of formula (II) are reacted with hydrogen (H₂). In one embodiment this includes employment of hydrogen or mixtures of hydrogen and at least one inert gas such as nitrogen or a noble gas. In a preferred embodiment hydrogen is employed.

The (partial) pressure of hydrogen (H₂) is typically from 10 hPa to 20 MPa, preferably from 500 hPa to 10 MPa, more preferably from 0.1 MPa to 8 MPa and yet even more preferably from 2 MPa to 8 MPa. Higher pressures are possible but to the best of applicant's knowledge do not add any advantage.

In an alternative embodiment the compounds of formula (II) are reacted with at least one hydrogen-carrier. As used herein hydrogen carrier is a compound that formally transfers hydrogen to compounds of formula (II). Suitable hydrogen carriers include isopropanol, cyclohexanol and formic acid. In this embodiment the hydrogen-carrier may act simultaneously as a solvent and as source of hydrogen. The ratio of compounds of formula (II) to the at least one hydrogen carrier is for example from 2:1 to 50:1, for example from 10:1 to 20:1.

In this alternative embodiment the process according to the invention is performed, for example, at a reaction temperature of 20 to 180°C, preferably 50 to 140°C.

The advantage of the present invention is that the process according to the invention allows to obtain the desired carboxylic acids of formula (I) with high selectivity in an efficient, highly robust process.

As a result of the process according to the invention, compounds of formula (I) or mixtures thereof are selectively obtained in good yields with very high space-time yields.

The reaction mixture may be then worked up, i.e. by separation of the compounds of formula (I) from the reaction mixture and/or their purification in a manner known to those skilled in the art.

Such separation and/or purification steps may encompass at least one process selected from the group consisting of filtration, centrifugation, sedimentation, drying, precipitation, extraction, crystallization, distillation and chromatographic processes.

The compounds of formula (I) may then be applied to manufacture downstream products like surfactants, cosmetics, paints, coatings, lubricants, adhesives, polymers and various other products.

The following examples are intended to illustrate the invention, but without limiting it thereto.

### Experimental part - Examples

### General

The Ruthenium on Carbon Catalyst (Ru/C) was commercially obtained from Strem (cat. no. 44-4065), with a Ruthenium content of 5 wt.-% and a specific surface area S_{BET} of 900 m²/g. The average particle size (d50) was 18 µm. Hydrogen (99.99 % purity) generated using a Hydrogen Generator H-Genie From Thales Nano was used for the reactions.

Zeolite H-ZSM5 Powder was prepared by calcination of a commercially available NH₄-ZSM5 precursor (Zeolite ZSM-5, ammonium Powder, S.A: 425 m²/g, 80:1 mole ratio SiO₂:Al₂O₃ from Thermo Fisher Scientific) by calcination at 550 °C for 5 hours.

Methyl 12-ketooctadecanoate was prepared via the oxidation and esterification of commercially available 12-hydroxystearic acid (purchased from Sigma Aldrich). 12-hydroxystearic acid was oxidized via NOCl in water and acetic acid, as per the literature method disclosed in US2019292134A1, to form 12-ketostearic acid, that was washed with water and dried under vacuum. Esterification of the resultant 12-ketostearic acid was conducted with H₂SO₄ in MeOH at 60 °C for one hour, then subsequent neutralization of the excess H₂SO₄ with Na₂CO₃. The solvent was removed *in-vacuo* and then methyl 12-ketooctadecanoate was isolated via extraction into CHCl₃ and removal of the solvent. The substrate was purified by MeOH recrystallisation and distillation in a kugelrohr distillation device (220 °C, 0.2 mbar).

### A) The reactor

The experiments were performed in a custom-made high pressure microreactors made out of stainless steel (V4A), equipped with a glass inlet. The reactor was fitted with a burst disk (80 bar), a pressure sensor and display, and a gas inlet and outlet, both controlled by needle valves. The reactions were stirred using magnetic stirring bars and a stirring plate.

### B) Product Analyses

The products were analyzed by ¹H NMR spectroscopy using a Bruker Avance III 400 MHz spectrometer. Residual proton solvent resonances were used as a reference and all chemical shifts (δ) are quoted in ppm and coupling constants in Hz. Multiplicity labels: s = singlet, d = doublet, t = triplet and m = multiplet.

The products were analyzed by gas chromatography with a mass-spectrometry detector. The instrument used was an Agilent 7890A gas chromatographer equipped with a Agilent mass detector 5975C (MSD). 0.2 µL of the sample were injected at 363 K and carried in a 1.2 mL/min helium flow through the column HP-5MS. The temperature of the column was constant at 363 K for 1 min and was then heated at 30 K/min up to 553K and finally held at 553K for 7 mins. The MS detector was fed by the helium stream air at 523K. The signal of each compound was calibrated and the calibration line used for quantification was determined by linear regression.

### Example 1: three-stage sequential reduction of methyl 12-ketooctadecanonate to methyl octadecenoate under neat conditions.

Methyl 12-ketooctadecanoate (200 mg) and Ru/C powder (20 mg) were added to a 16 ml vial with a magnetic stirrer bar and the vial was inserted into the high pressure microreactor and the reactor closed tight. The reactor was pressurized with 2.5 MPa H₂ and heated to 165 °C and the reaction was stirred at 1000 rpm. After three hours of reaction time, the reactor was cooled to room temperature and the vial was removed. The product was isolated as a waxy solid by methanol extraction with a 96% yield. The conversion was determined by ¹H NMR spectroscopy, with the following characteristic signals of 12-hydroxyoctadecanoate with essentially quantitative conversion:
¹H NMR (CDCl₃, 400 MHz): δ 3.66 (s, OCH₃, 3H), 3.57 (m, C*H*OH, 1H), 2.30 (t, *J_{HH}* = 7.6 Hz, CH₂COOCH₃, 2H), 1.61 (m, C*H*₂CH₂COOCH₃, 2H), 1.51-1.35 (m, in-chain CH₂, 6H), 1.35-1.22 (m, in-chain CH₂, 20H), 0.87 (t, *J_{HH}* = 7.3 Hz, CH₃, 3H).

In the second step. The 12-hydroxyoctadecanoate from the first step was added to a 16 ml vial (200 mg) with Zeolite H-ZSM5 (10 mg, 80:1 mole ratio SiO₂:Al₂O₃) and a stirrer bar. The reaction mixture was stirred at 1000 rpm and heated to 224 °C. After two hours at 224 °C, the vial was cooled to room temperature and the product was removed by methanol extraction with a 90% yield. The while oily product was characterized by ¹H NMR spectroscopy with essentially quantitative conversion of the starting material:
¹H NMR (CDCl₃, 400 MHz): δ 5.37 (m, HC=CH, 2H), c3.66 (s, OCH₃, 3H), 2.30 (t, *J_{HH} =* 7.6 Hz, CH₂COOCH₃, 2H), 1.98 (m, CH₂-CH=CH-C*H*₂, 4H), 1.61 (m, C*H*₂CH₂COOCH₃, 2H), 1.37-1.21 (m, in-chain CH₂, 20H), 0.87 (t, *J_{HH} =* 7.3 Hz, CH₃, 3H).

In the third step, the product from the second step was added to a 16 ml vial (163 mg) with Ru/C (16 mg) and a stirrer bar. The vial was added to a high pressure microreactor and the reactor was closed. The reactor was pressurized to 4.0 MPa H₂ and heated to 230 °C. After four hours, the reactor was cooled to room temperature and the product was isolated via a methanol extraction and solvent removal, giving a colourless oil in a 99% yield. The conversion was determined by ¹H NMR spectroscopy, and was essentially quantitative:
¹H NMR (CDCl₃, 400 MHz): δ 3.66 (s, OCH₃, 3H), 2.30 (t, *J_{HH} =* 7.6 Hz, CH₂COOCH₃, 2H), 1.98 (m, CH₂-CH=CH-C*H*₂, 4H), 1.62 (pentet, *J_{HH} =* 7.6) C*H*₂CH₂COOCH₃, 2H), 1.35-1.20 (m, in-chain CH₂, 28H), 0.87 (t, *J_{HH}* = 7.3 Hz, CH₃, 3H).

### Example 2: single-stage complete reduction of methyl 12-ketooctadecanonate to methyl octadecenoate under neat conditions.

Methyl 12-ketooctadecanoate (200 mg), Ru/C powder (20 mg) and Zeolite H-ZSM5 (15 mg, 80:1 mol ratio of SiO₂:Al₂O₃) were added to a 16 ml vial with a magnetic stirrer bar and the vial was inserted into the high pressure microreactor and the reactor closed tight. The reactor was pressurized with 3.5 MPa H₂ and heated to 182 °C and the reaction was stirred at 1000 rpm. After three hours of reaction time, the reactor was cooled to room temperature and the vial was removed. The product was isolated as a colourless oil by methanol extraction and solvent removal with a 70% yield. The conversion was determined by ¹H NMR spectroscopy, with the following characteristic signals of methyl octadecenoate:
¹H NMR (CDCl₃, 400 MHz): δ 3.66 (s, OCH₃, 3H), 2.30 (t, *J_{HH} =* 7.6 Hz, CH₂COOCH₃, 2H), 1.98 (m, CH₂-CH=CH-C*H*₂, 4H), 1.62 (pentet, *J_{HH} =* 7.6) C*H*₂CH₂COOCH₃, 2H), 1.35-1.20 (m, in-chain CH₂, 28H), 0.87 (t, *J_{HH}* = 7.3 Hz, CH₃, 3H).

## Claims

1. A process for the preparation of compounds of formula (I)
[EG-R-COO]ₙA (I),
wherein
n is 1, 2 or 3, preferably 1 or 3, more preferably 1,
A is
for n=1 hydrogen or C₁-C₁₂-alkyl, preferably hydrogen or C₁-C₄-alkyl, more preferably hydrogen, methyl or ethyl,
for n=2 C₂-C₆-alkanediyl, preferably 1,2-ethandiyl and
for n=3 1,2,3-propanetriyl
EG is an end group selected from COOE, CH=O, CH₂-OH and CH₃, preferably selected from COOE, CH₂-OH and CH₃ and in one embodiment from COOE and CH₂-OH, wherein E is hydrogen or C₁-C₁₂-alkyl, preferably hydrogen or C₁-C₄-alkyl, more preferably hydrogen, methyl or ethyl
R is C₆-C₄₈-alkanediyl, preferably C₆-C₄₀-alkanediyl and even more preferably C₆-C₃₂-alkanediyl
the process comprising at least the steps of reacting compounds of formula (II)
[EG-R_{OX}-COO]ₙA (II),
wherein n, A and EG have the meaning defined above for formula (I) including their preferences and
Rox is C₆-C₄₈-alkanediyl or C₆-C₄₈-alkenediyl, which is once or more substituted by substituents selected from hydroxy and/or oxo, preferably C₆-C₄₀-alkanediyl or C₆-C₄₀-alkenediyl, which is once or more substituted by substituents selected from hydroxy and/or oxo and even more preferably C₆-C₃₂-alkanediyl or C₆-C₃₂-alkenediyl which is once or more substituted by substituents selected from hydroxy and/or oxo
either sequentially or simultaneously, preferably simultaneously
• with molecular hydrogen (H₂) or a hydrogen carrier in the presence of at least one hydrogenation catalyst comprising a transition metal or a salt thereof whereby the transition metal is selected from Ni, Cu, Mo, Cr, W, Ru, Rh, Pt, Pd and Ir and
• at least one dehydration catalyst.

2. The process according to claim 1, wherein specific compounds of formula (I) are those of formula (Ia)
[EOOC-R-COO]ₙA (Ia),
wherein R, E, n and A have the meaning as defined above for formula (I) in claim 1
by reacting as specific compounds of formula (II) those of formula (IIa) [E€OOC-R_{OX}-COO]ₙA (IIa),
wherein Rox has the meaning as defined above for formula (I) in claim 1.

3. The process according to claim 1 or 2, wherein the process is performed such that mixtures comprising at least two, preferably at least five and more preferably at least ten different compounds of formula (II) are employed.

4. The process according to any one of claims 1 to 3, wherein the process is performed such that mixtures of compounds of formula (II) having at least two, preferably at least five and more preferably at least ten, for example ten to fourty-three different numbers of carbon atoms are employed.

5. The process according to any one of claims 1 to 4, wherein the compounds of formula (II) are employed in neat form or in a solvent that is inert or virtually inert under the reaction conditions.

6. The process according to any one of claims 1 to 5, wherein mixtures of formula (IIa) as defined in claim 2 are employed.

7. The process according to claim 6, wherein the mixtures of starting materials of formula (IIa) as defined in claim 2 are prepared by at least the step of reacting polyethylene having a number-average molecular weight Mₙ of at least 20,000 g/mol with an oxygen-containing gas at a temperature of from the melting point of the polyethylene to 300°C in the presence of an oxidation catalyst, for example an oxidation catalyst that is insoluble in the reaction mixture.

8. The process according to any one of claims 1 to 7 wherein the at least one hydrogenation catalysts is selected from the group of transition metals Ni, Cu, Mo, Cr, W, Ru, Rh, Pt, Pd and Ir or a salt thereof which is supported on a carrier material such as zirconium dioxide, titanium dioxide, silicates such as magnesium or aluminium silicates, aluminium oxides, silicon dioxide, graphite, activated carbons or alkaline earth metal compounds as well as on mixtures of these carrier materials.

9. The process according to any one of claims 1 to 8 wherein the at least one dehydration catalyst is selected from the group of metal oxide catalysts of chromium, molybdenum or tumgsten; zeolites, aluminas and heteropoly acid salts wherein the protons in the heteropoly acids are partially or fully exchanged with metal cations.

10. The process according to any one of claims 1 to 9, wherein the at least one hydrogenation catalyst and the at least one dehydration catalyst are selected from Ruthenium on H-ZSM5; Nickel and Cobalt on H-ZSM5 or PtWO₃ supported on alumina which show both required activities.

11. The process according to any one of claims 1 to 10, wherein the process is performed batchwise or continuously.

12. The process according to any one of claims 1 to 11, wherein the reaction temperature is 20 to 280°C, preferably 100 to 250°C, more preferably 180 to 220 °C.

13. The process according to any one of claims 1 to 12, wherein the compounds of formula (II) are reacted with hydrogen (H₂), for example with a partial pressure from 10 hPa to 20 MPa, preferably from 500 hPa to 10 MPa, more preferably from 0.1 MPa to 8 MPa and yet even more preferably from 2 to 8 MPa.

14. The process according to any one of claims 1 to 13, wherein the compounds of formula (II) are reacted with a hydrogen carrier, which is preferably selected from the group consisting of isopropanol, cyclohexanol and formic acid.

15. A process for the manufacturing of surfactants, cosmetics, paints, coatings, lubricants, adhesives and polymers including compounds of formula (I) obtained according to a process according to any one of claims 1 to 14.
